# DEMANDE DE BREVET EUROPEEN

(11) **EP 0 922 436 A1**
(43) Date de publication de la demande: **16.06.1999**
(21) Numéro de dépôt: 98420230.9
(22) Date de dépôt: 10.12.1998
(51) Int. Cl.: A61B 17/68, A61F 2/08, A61F 2/28

(54) **Agrafe d'ostéosynthèse, guide de pose et kit d'ostéosynthese**

(30) Priorité: 10.12.1997 FR 9715897
(71) Demandeur: Memometal Industries, 73220 Aiton (FR)
(72) Inventeur: Prandi, Bernard, 74210 Seythenex (FR); Giquel, Loic, 35600 Dinard (FR); Graf, Patrice, 29820 Bohard (FR); Bonfiglio, Giuseppe, 20040 Bellusco (IT); Rinaldi, Giulio, 20152 Milan (IT); Fascia, Michele, 20148 Milan (IT); Salina, Carlo, Aicurzio (IT)
(74) Mandataire: Myon, Gérard Jean-Pierre

(57) **Abrégé**

L'agrafe de l'invention est constituée par une canule (2) en matériau malléable qui peut être écrasée et appliquée à la périphérie de l'os (1).

Une broche rigide (3) apte à être coiffée par la canule (2) est utilisée pour la mise en place de l'extrémité antérieure (2b) de la canule dans l'os.

Application à la réduction de la fractures, notamment de fractures multi-esquilleuses.

## Description

L'invention a trait à une agrafe d'ostéosynthèse, à un guide de pose d'une telle agrafe et à un ensemble ou kit d'ostéosynthèse .

Il est connu d'utiliser une agrafe d'ostéosynthèse pour la chirurgie de consolidation de fractures osseuses chez un patient humain ou animal. Par la demande de brevet français 2 694 696, on connaît une agrafe d'ostéosynthèse réalisée en alliage à transition austénite-martensite. Une telle agrafe a une forme globale en U et comprend deux branches destinées à pénétrer respectivement dans les deux parties d'un os a solidariser. Bien que présentant des avantages certains, cette agrafe ne peut pas être utilisée pour tous les os, et en particulier dans le cas de fractures multi-esquilleuses, d'une part car elle ne permet pas de plaquer une esquille contre la partie mère de l'os et, d'autre part, en raison des dimensions trop faibles et du trop grand nombre d'esquilles à solidariser.

Pour la réduction de fractures de ce type, on peut utiliser une broche de Kirschner, rigide et pleine, perforer les parties osseuses à solidariser et utiliser cette broche comme système de fixation en courbant l'extrémité qui dépasse de l'os à la manière d'un clou pour éviter toute migration vers l'extérieur des fragments d'os. Cependant, du fait de la rigidité d'une telle broche qui est pleine, l'opération de courbure ou de pliage de l'extrémité de la broche qui dépasse induit sur l'extrémité de la broche qui est plantée dans l'os un moment important qui a pour effet de déchausser au moins partiellement cette broche. En outre, les broches de ce type sont prévues pour être normalement retirées de l'os après une période variable de l'ordre de six semaines, ce qui nécessite une seconde opération. Enfin, compte tenu de la rigidité des broches pleines, il n'est en général pas possible de faire plaquer l'extrémité de la broche qui dépasse des fragments osseux sur l'os cortical, de sorte que l'extrémité libre de la broche peut interférer avec les os, les tissus ou les ligaments voisins.

C'est à ces inconvénients qu'entend plus particulièrement remédier l'invention en proposant une agrafe d'osteosynthèse qui peut être utilisée pour la fixation et la consolidation de fractures multi-esquilleuses sans présenter les inconvénients des broches précitées.

Dans cet esprit, l'invention concerne une agrafe d'ostéosynthèse caractérisée en ce qu'elle est constituée par une canule en matériau malléable.

Grâce à l'invention, le fait que l'agrafe est creuse permet de conformer facilement la partie de l'agrafe qui dépasse des fragments osseux pour épouser précisément la surface extérieure de ces fragments. En outre, le caractère creux et malléable de la canule permet de le conformer en exerçant sur celle-ci des efforts relativement faibles, ce qui limite les risques de déplacement des fragments et de déchaussement. Enfin, une migration d'os spongieux peut avoir lieu dans l'extrémité creuse de la canule lors de sa mise en place, ce qui améliore l'ancrage de l'agrafe dans celui-ci.

Les indications de l'agrafe de l'invention sont nombreuses et concernent l'ensemble de la chirurgie traumatologique et orthopédique, humaine ou vétérinaire. On peut citer l'ostéosynthèse de fragments osseux de petites et moyennes dimensions, la fixation de greffes osseuses, l'ostéosynthèse de désinsertion tendineuse avec fragments osseux, le traitement des fractures plus ou moins comminutives ou multi-esquilleuses, la fixation de petites ostéotomies, la fixation de fractures-arrachements chondro-éphysaires, la reconstruction ligamentaire, notamment la chirurgie du ligament croisé antérieur, etc...

Selon un premier aspect avantageux de l'invention, la canule comprend une extrémité biseautée destinée à pénétrer dans un os du patient. Le caractère biseauté de cette extrémité facilite sa pénétration et son remplissage d'os spongieux en vue d'une meilleure immobilisation de cette extrémité.

Selon un autre aspect de l'invention, on peut prévoir qu'une extrémité de la canule est arrondie. Ceci permet de limiter les risques d'agression pour les tissus voisins lorsque la canule transperce un os.

Selon un autre aspect de l'invention, la canule est réalisée en acier inoxydable recuit, ce qui garantit sa biocompatibilité, son caractère solide et sa malléabilité.

Avantageusement, la canule à un diamètre extérieur compris entre 1 et 5 mm, de préférence de entre 1 et 3 mm et un diamètre intérieur compris entre 0,5 et 4,5 mm de préférence entre 1,5 et 2,5 mm.

L'invention concerne également un guide de pose d'une agrafe d'ostéosynthèse telle que précédemment décrite qui comprend une broche rigide apte à être coiffée par la canule. Cette broche a pour effet de rigidifier la canule formant l'agrafe lors de son insertion dans l'os à consolider.

Selon un aspect avantageux, cette broche est pourvue d'une extrémité à plusieurs faces, de telle sorte qu'elle est apte à servir de foret.

Selon un autre aspect avantageux, cette broche est réalisée en alliage super-élastique à base de titane, ce qui lui confère d'excellentes propriétés mécaniques.

L'invention concerne enfin un kit d'ostéosynthèse formé d'au moins une agrafe et d'au moins une broche telles que précédemment décrites. Un tel kit permet de traiter efficacement des fractures, notamment multi-esquilleuses. Si le kit comprend plusieurs canules et broches, celles-ci peuvent avoir des dimensions différentes, pour que le kit soit efficace vis-à-vis de fractures de type et de configuration différentes, ou des extrémités de géométries différentes.

L'invention sera mieux comprise et d'autres avantages de celle-ci apparaîtront plus clairement à la lumière de la description qui va suivre d'une agrafe d'ostéosynthèse conforme à son principe et de sa méthode de pose, donnée uniquement à titre d'exemple et faite en référence aux dessins annexés dans lesquels :
- la figure 1 montre en perspective un kit d'ostéosynthèse selon l'invention ;
- la figure 2 est une coupe longitudinale de l'extrémité d'un os et d'une agrafe conforme à l'invention, lors d'une première étape de pose ;
- la figure 3 est une vue analogue à la figure 2 lors d'une seconde étape de pose ;
- la figure 4 est une vue analogue à la figure 2 lors d'une troisième étape de pose et
- la figure 5 est une vue analogue à la figure 2 au terme de la pose.

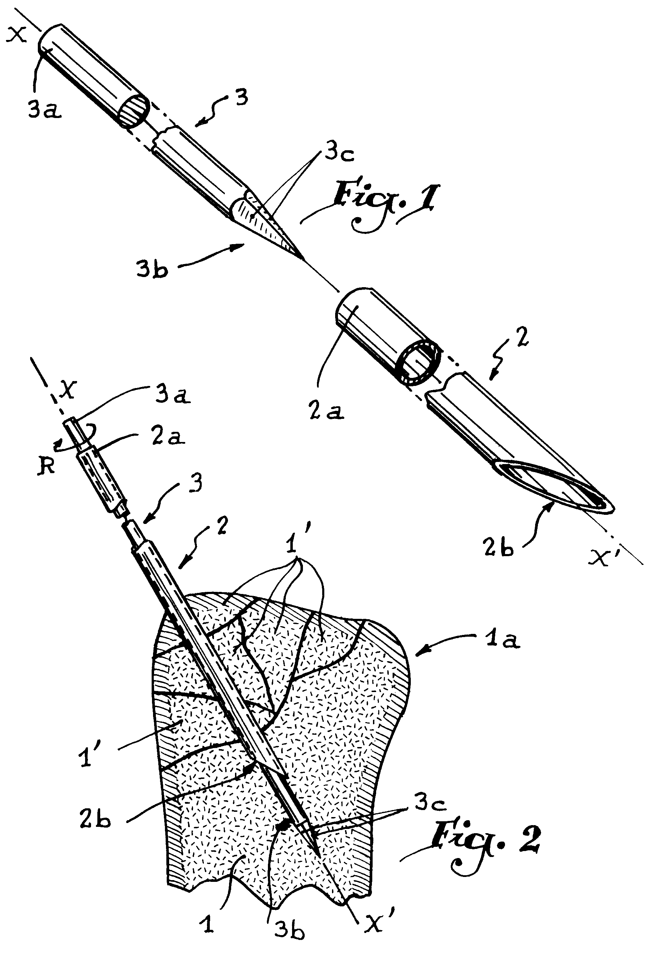

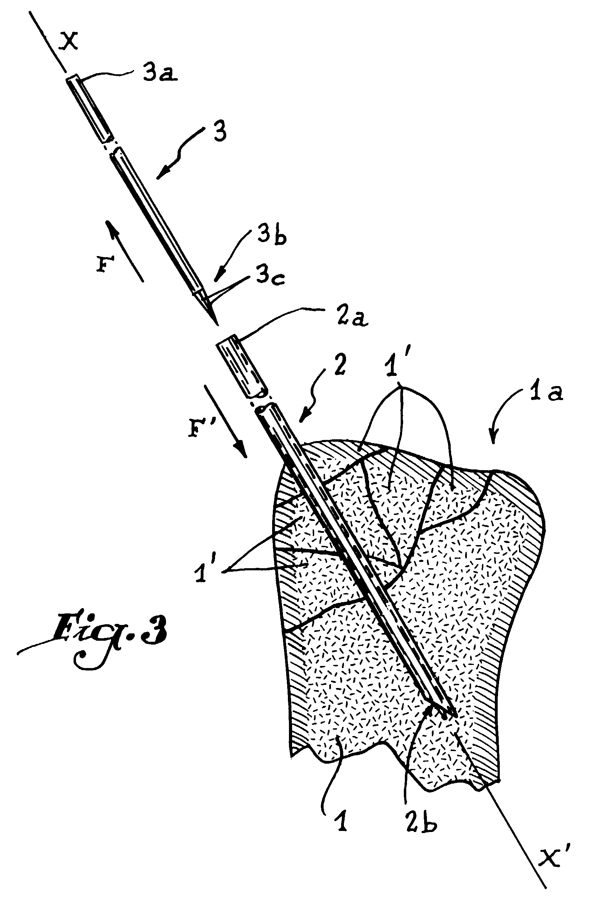

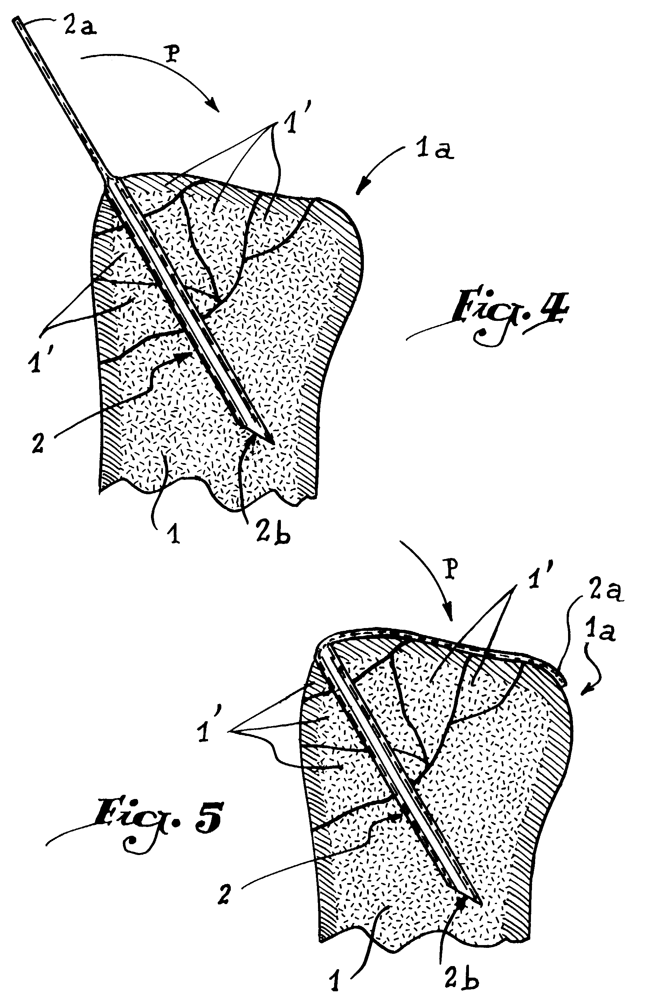

A la figure 2, un os 1 est représenté brisé au niveau de son extrémité 1a qui est divisée en plusieurs fragments 1'. Une agrafe d'ostéosynthèse représentée à la figure 1 est constituée par une canule ou tube 2 à l'intérieur de laquelle ou duquel peut être glissée une broche rigide 3, de telle sorte que la broche 3 entourée de la canule 2 peut être introduite dans la partie principale de l'os 1 à travers les fragments 1', comme cela est représenté aux figures 2 à 5.

L'extrémité arrière 3a de la broche 3 peut être montée sur le mandrin d'une perceuse qui l'entraîne en rotation autour de son axe de symétrie XX', comme représenté par la flèche R à la figure 2. L'extrémité avant 3b de la broche 3 est conformée en pointe et comprend quatre faces sensiblement triangulaires 3c, de telle sorte que la broche 3 joue le rôle d'un foret lors de son introduction dans l'os.

Lorsque l'ensemble ou kit formé de la canule 2 et de la broche 3 est assez profondément enfoncé dans l'os 1 pour que l'extrémité avant 3b de la broche 3 pénètre dans la partie non brisée de l'os, le mouvement de rotation est arrêté et la broche 3 est retirée comme cela est représenté par la flèche F à la figure 3.

Comme la canule 2 est disposée autour de la broche 3 lors de son introduction dans l'os, la canule reste en place à travers les fragments 1' et jusque dans la partie saine de l'os 1. Elle est alors dans la position de la figure 3. En exerçant un effort de poussée F' sur son extrémité postérieure 2a, on enfonce la canule 2 jusqu'au fond du trou réalisé grâce à la broche 3. On note que l'extrémité antérieure 2b de la canule 2 est biseautée, de sorte que ceci facilite sa pénétration dans l'os au fond du perçage réalisé grâce à la broche 3.

Une partie de l'os spongieux et des fragments créés par les faces 3c de la broche 3 pénètre à l'intérieur de l'extrémité 2b de la canule 2 et s'y accumule. Ceci a pour effet de faciliter l'ancrage de la canule 2 dans l'os 1.

Selon une variante non représentée de l'invention, il serait également possible de mettre en place la broche 3 dans l'os 1 alors que celle-ci n'est pas coiffée par la canule 2, puis, lorsque la broche 3 est suffisamment enfoncée dans l'os 1, de faire glisser la canule 2 autour de la broche 1 dans l'os, eventuellement en impactant la canule. Il serait également possible de retirer la broche 3 de l'os 1, puis d'introduire l'extrémité avant de la canule 2 dans le trou foré grâce à la broche 3. Dans tous les cas, on atteint la position de la figure 3.

Il est alors possible d'écraser, à l'aide d'une pince, l'extrémité postérieure 2a de la canule 2 qui dépasse des fragments osseux 1'. Cet écrasement est effectué jusqu'au plus près possible des fragments osseux afin de les immobiliser au mieux. Cette opération est réalisée grâce au fait que le matériau constitutif de la canule 2 est malléable. Un matériau convenable est en acier inoxydable tel que de l'acier 316 LUM.

Selon une variante non représentée de l'invention, l'extrémité postérieure 2a peut être coupée, avant ou après l'opération d'écrasement, pour ajuster la longueur de la canule 2 aux dimensions de l'os.

Comme le matériau constitutif de la canule 2 est malléable, les efforts exercés sur l'extrémité 2b de la canule 2 lors de l'écrasement de l'extrémité 2a sont faibles, voire négligeables, de sorte qu'il y a peu de risque de déplacer la canule 2.

Il est alors possible de rabattre l'extrémité 2a contre l'os, notamment contre l'un des fragments osseux 1', car l'écrasement de l'extrémité 2a l'a rendue souple dans une direction perpendiculaire à l'axe XX' de la partie enfoncée du tube 2. Ceci est représenté par la flèche de pliage P à la figure 4. Comme représenté par la flèche de pliage P à la figure 5, l'extrémité 2a peut être plaquée précisément contre les fragments 1' au point qu'elle ne dépasse pas sensiblement de la surface de l'os reconstitué, ce qui évite tout danger d'accrochage avec des os, des muscles ou des ligaments voisins.

De fait du caractère malléable de la canule 2, on peut également envisager de corriger le placement de l'extrémité 2a après une première tentative. Ceci permet au chirurgien de plaquer l'extrémité 2a sur des fragments osseux différents pour apprécier dans quelle configuration la canule 2 est dans une position optimale sur le plan anatomique.

En d'autres termes, le pliage de la canule après le retrait de la broche permet d'ajuster la configuration de l'agrafe de l'invention sur la géométrie de l'os cortical sans déplacer les fragments osseux.

Selon une variante non représentée de l'invention, l'extrémité antérieure de la canule 2 peut être arrondie, notamment si elle risque de transpercer l'os de part en part. Ceci permet de limiter les risques d'agression des tissus et os voisins. Dans ce cas, les deux extrémités de la canule sont écrasées avant d'être plaquées contre l'os cortical comme indiqué précédemment en référence à l'extrémité 2a.

Dans tous les cas, l'immobilisation réalisée grâce au kit d'ostéosynthèse de l'invention est d'excellente qualité, elle permet une meilleure consolidation sans nécessiter une immobilisation plâtrée, à l'exception d'une immobilisation de courte durée en cas d'oedème important. En outre, la stabilité et la dynamique du montage réalisé grâce à l'invention favorise la formation du cal osseux. Enfin, du fait de la souplesse de la canule ou tube 2, il n'existe pas de fragilisation de l'os cortical.

On comprend qu'il est aisé d'adapter la longueur et/ou le diamètre de la canule 2 et de la broche 3 en fonction du type d'os sur lequel doit être pratiquée une intervention. En pratique, des canules dont le diamètre extérieur est compris en 1 et 5 mm et dont le diamètre intérieur est compris entre 0,5 et 4,5 mm ont donné des résultats satisfaisants. On pourra préférer des canules dont le diamètre extérieur est compris entre 1 et 3 mm alors que leur diamètre intérieur est compris entre 1,5 et 2,5 mm.

Avantageusement, la broche 3 est réalisée en alliage de titane super-élastique, ce qui lui confère une bonne rigidité et une souplesse appréciable, notamment lorsque, en cours d'introduction, la tige est déviée, ce qui résulte dans une courbure de celle-ci et de la canule. En effet, le caractère super-élastique de la broche 3 permet de la retirer aisément de l'os même lorsqu'elle est courbée. Un alliage de 44,5 ± 0,5% de titane et de 55,5 ± 0,5% de nickel en poids donne des résultats particulièrement satisfaisants.

On comprend que, du fait de sa bonne adaptabilité à la morphologie du patient, l'agrafe de l'invention peut être laissée en place et ne nécessite donc aucune intervention ultérieure pour son retrait.

L'invention a été représentée avec une canule à section circulaire. Elle est cependant applicable avec des canules à section ovale ou polygonale.

## Revendications

1. Agrafe d'ostéosynthèse, caractérisée en ce qu'elle est constituée par une canule (2) en matériau malléable.

2. Agrafe d'ostéosynthèse selon la revendication 1, caractérisée en ce que ladite canule (2) comprend une extrémité biseautée (2b) destinée à pénétrer dans un os (1) du patient.

3. Agrafe d'ostéosynthèse selon la revendication 1, caractérisée en ce que ladite canule comprend une extrémité arrondie.

4. Agrafe d'ostéosynthèse selon l'une des revendications 1 à 3, caractérisée en ce que ladite canule (2) est réalisée en acier inoxydable récuit.

5. Agrafe d'ostéosynthèse selon l'une des revendications précédentes, caractérisée en ce que ladite canule (2) a un diamètre extérieur compris entre 1 et 5 mm, de préférence entre 1 et 3 mm.

6. Agrafe d'ostéosynthèse selon l'une des revendications précédentes, caractérisée en ce que ladite canule a un diamètre intérieur compris entre 0,5 et 4,5 mm, de préférence entre 1,5 et 2,5 mm.

7. Guide de pose d'une agrafe d'ostéosynthèse selon l'une des revendications précédentes, caractérisé en ce qu'il comprend une broche rigide (3) apte à être coiffée par ladite canule (2).

8. Guide de pose selon la revendication 7, caractérisé en ce que ladite broche (3) est pourvue d'une extrémité (3b) à plusieurs faces (3c).

9. Guide de pose selon la revendication 7, caractérisé en ce que ladite broche (3) est réalisée en alliage super-élastique à base de titane.

10. Kit d'ostéosynthèse, caractérisé en ce qu'il comprend au moins une agrafe selon l'une des revendications 1 à 6 et au moins un guide selon l'une des revendications 7 à 9.
